# EUROPEAN PATENT SPECIFICATION

(11) **EP 4 282 452 B1**
(45) Date of publication and mention of the grant of the patent: **10.04.2024**
(21) Application number: 22174992.2
(22) Date of filing: 24.05.2022
(51) Int. Cl.: A61M 15/00, A61B 5/087

(54) **NEBULIZER WITH DETECTING STRUCTURE**
ZERSTÄUBER MIT DETEKTIONSSTRUKTUR
NÉBULISEUR POURVU DE STRUCTURE DE DÉTECTION

(43) Date of publication of application: 29.11.2023
(73) Proprietor: Galemed Corporation, Yilan County 268 (TW)
(72) Inventor: CHEN, Po-Chang, 268 Yilan County (TW); WANG, Hsin-Chen, 268 Yilan County (TW); YANG, Chia-Chin, 268 Yilan County (TW); CHEN, Hao-Hsiang, 268 Yilan County (TW); HSU, Chun-Wei, 268 Yilan County (TW)
(74) Representative: 2K Patentanwälte Blasberg Kewitz & Reichel

(56) References cited:
- US-A1- 2017 296 772
- US-A1- 2018 008 790
- US-A1- 2018 161 531
- US-A1- 2021 299 366

## Description

### BACKGROUND OF THE DISCLOSURE

### Technical Field

The technical field relates to a nebulizer, and more particularly relates to a nebulizer with a detecting structure.

### Description of Related Art

A nebulizer is a medical device that administers medicine through the respiratory system. The nebulizer atomizes the liquid medicine into tiny liquid droplets with a certain particle size. The atomized medicine is administered into the patient's circulatory system by the nasal and oral administration through inhalation and respirations to accomplish treatment.

However, when using existing nebulizers, most of the nebulizers continuously atomize the liquid medicine without taking a patient's respiratory rate into account during the atomizing process of the liquid medicine, and the nebulizers continuously administer the atomized medicine while the user exhales, and that decreases the effect of administering medication and may cause discomfort to the user.

In view of the above drawbacks, the inventor proposes this disclosure based on his expert knowledge and elaborate researches in order to solve the problems of related art.

US 2018/161531 A1 discloses a nebulizer according to the preamble of claim 1. The nebulizer monitors the inhalation and exhalation flow generated by the patient and communicates proper breathing technique for optimal drug delivery. The nebulizer system may monitor air supply to the nebulizer to ensure it is within the working range and is producing, or is capable of producing, acceptable particle size and drug output rate.

US 2021/299366 A1 discloses an breathing airflow detecting device for a nebulizing device, which includes a case, a light switching module and a light blocking element being arranged in the case. The light blocking element is arranged between optical routes of a light emitting element and a light receiving element. When airflow from an inlet blows and swings the light blocking element, the light blocking element blocks light or passes light to the light receiving element. When the light receiving element detects the variation of light, the light receiving element emits a signal from the output port of the light switching module to drive or stop driving a nebulizing device. As a result, when a patient breathes, the nebulizing device is driven such that the volume of the nebulized medicine is accurately inhaled into the patient's lungs.

US 2018/008790 A1 discloses an oscillating positive expiratory pressure system including an oscillating positive expiratory pressure device having a chamber, an input component in communication with the chamber, wherein the input component is operative to sense a flow and/or pressure and generate an input signal correlated to the flow or pressure.

US 2017/296772 A1 discloses a respiratory care system including a user interface. A flow indicator is movable in response to inhalation and/or exhalation, or both, by a user through the user interface. An electronic indicator is operable in response to an electronic signal transmitted in response to the movement of the floe indicator.

### SUMMARY OF THE DISCLOSURE

It is an object of the present disclosure to provide an improved nebulizer with the detecting structure disposed in the nozzle tube for detecting the user's exhalation with a simple set-up.

This problem is solved by a nebulizer as claimed in claim 1. Further advantageous embodiments are the subject-matter of the dependent claims.

According to the present invention, the nebulizer stops atomization of the liquid medicine during the user's exhalation to avoid wasting the liquid medicine, and the effect of administering medication may be improved.

In comparison with the related art, the nebulizer in this disclosure includes the detecting structure disposed in the nozzle tube to detect the user's exhalation. When the user exhales, the air blown from the nozzle flows into the tube and blows the swinging member to drive the shutter to activate the sensor. Furthermore, the sensing signal sent from the sensor is transmitted to the control module to achieve the purpose of detecting the user's exhalation. Therefore, the nebulizer stops atomizing the liquid medicine to avoid wasting the liquid medicine and improves the effect of administering medication.

### BRIEF DESCRIPTION OF THE DRAWINGS

The features of the disclosure believed to be novel are set forth with particularity in the appended claims. The disclosure itself, however, may be best understood by reference to the following detailed description of the disclosure, which describes a number of exemplary embodiments of the disclosure, taken in conjunction with the accompanying drawings, in which:
FIG 1 is a perspective schematic view of the nebulizer with a detecting structure in this disclosure.
FIG 2 is a perspective exploded view of the nebulizer with a detecting structure in this disclosure.
FIG 3 is a perspective exploded view of the nozzle tube in this disclosure.
FIG 4 is a perspective schematic view of the nozzle tube in this disclosure.
FIG 5 is a cross sectional view of the nozzle tube in this disclosure.
FIG 6 is a schematic view illustrating operation of the nebulizer in exhalation in this disclosure.
FIG 7 is a schematic view of the air flow path of the nebulizer in exhalation in this disclosure.
FIG 8 is a cross sectional view of the nebulizer in exhalation in this disclosure.

### DETAILED DESCRIPTION

The technical contents of this disclosure will become apparent with the detailed description of embodiments accompanied with the illustration of related drawings as follows. It is intended that the embodiments and drawings disclosed herein are to be considered illustrative rather than restrictive.

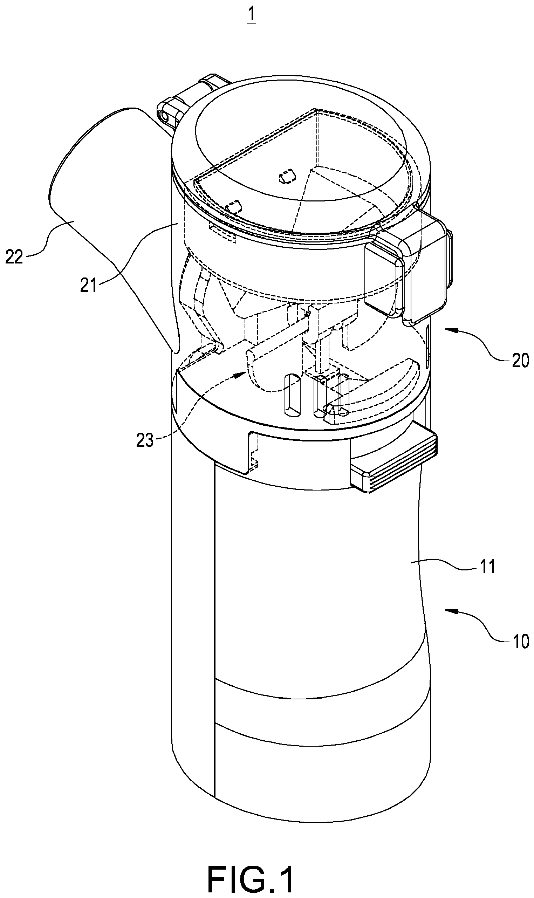

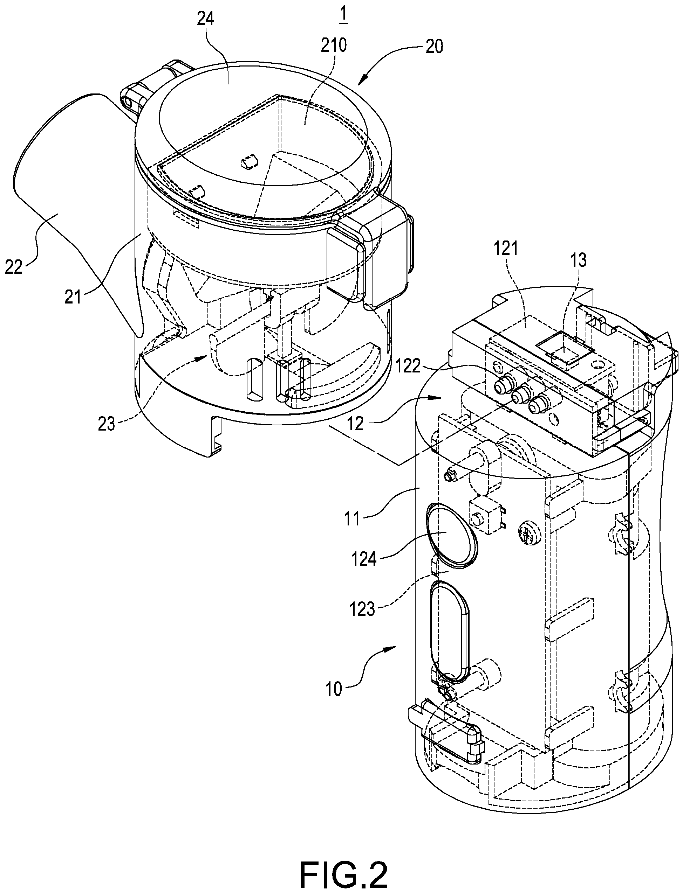

Please refer to FIG 1 and FIG 2, which depict a perspective schematic view and a perspective exploded view of the nebulizer with a detecting structure in this disclosure. The nebulizer 1 with a detecting structure in this disclosure includes a host 10 and a nozzle tube 20. The nozzle tube 20 is combined on the host 10. The nozzle tube 20 is used to accommodate and atomize the liquid medicine to provide the aerosolized liquid medicine for the user through inhalation. It should be noted that a detecting structure is disposed in nozzle tube 20 for detecting the user's exhalation. Thus, the nebulizer may stop atomizing the liquid medication during the user's exhalation to avoid wasting liquid medicine and improve the effect of administering medication.

The host 10 includes a main body 11, a control module 12 disposed in the main body 11 and a sensor 13 electrically connected with the control module 12. In this embodiment, the control module 12 includes a sensing circuit board 121 and a plurality of conductive terminals 122. The sensor 13 is arranged on the sensing circuit board 121 and electrically connected to the conductive terminals 122. Furthermore, the control module 12 further includes a controlling circuit board 123 and a plurality of buttons 124. The controlling circuit board 123 is electrically connected to the plurality of buttons 124 and the sensing circuit board 121.

The nozzle tube 20 includes a tube 21, a nozzle 22 and a detecting structure 23 disposed in the tube 21. The tube 21 includes a chamber 210 for accommodating the liquid medicinal. In this embodiment, the nozzle tube 20 further includes a top cover 24, and the top cover 24 is movably combined with the tube 21 and covers the chamber 210. In some embodiments, the user may open the top cover 24 and fill the liquid medicine into the chamber 210 for atomizing. Then, the user may inhale the atomized liquid medicine in the chamber 210 from the nozzle 22. The structure of the nozzle tube 20 is described in more detail as follows.

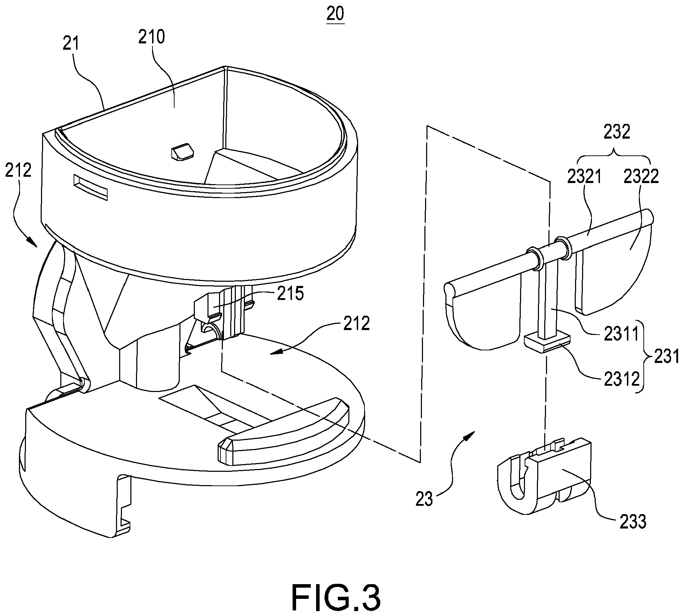

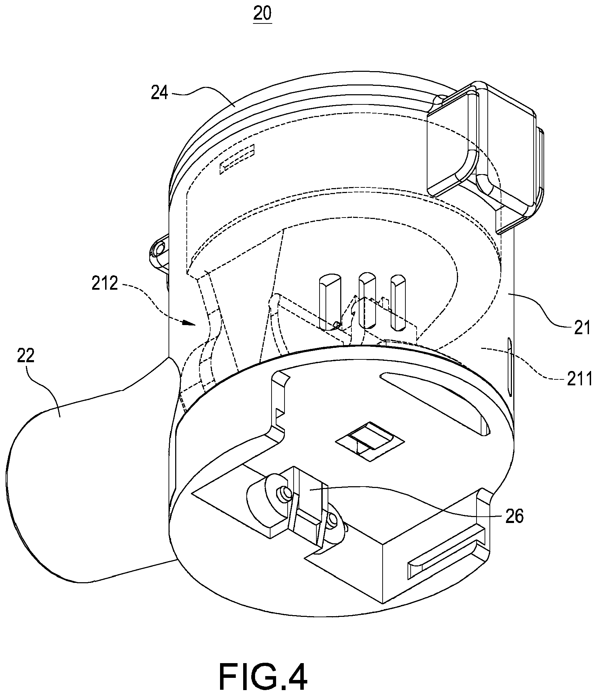

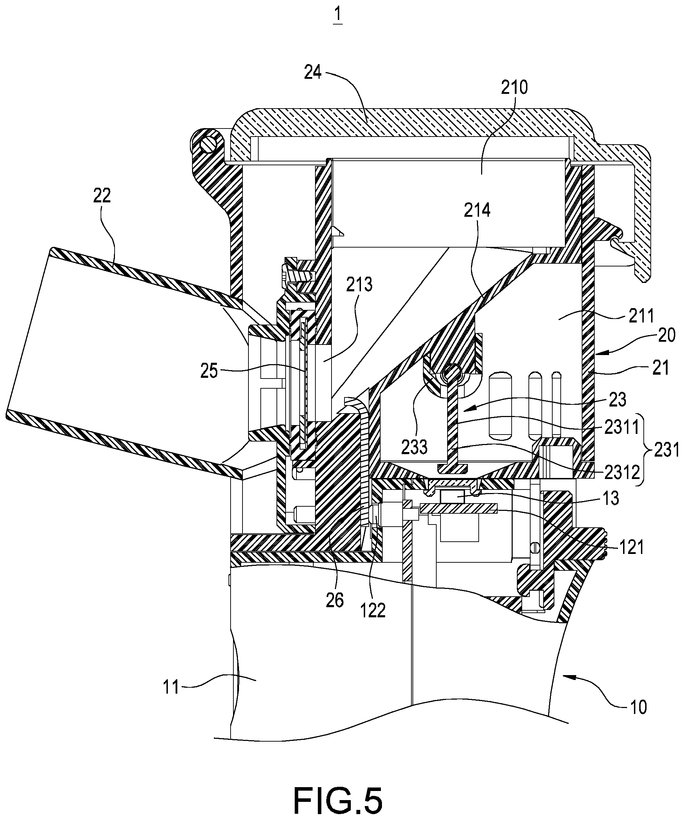

Please further refer to FIG 3 to FIG 5, which depict a perspective exploded view, a perspective schematic view and a cross sectional view of the nozzle tube in this disclosure. As shown in FIG 3, the tube 21 includes a chamber 210, an exhaust chamber 211, an air passage 212 and a liquid medicine releasing hole 213. The detecting structure 23 is arranged in the exhaust chamber 211. The nozzle 22 is arranged on one side of the tube 21 and communicates with the chamber 210. The nozzle 22 communicates with the exhaust chamber 211 through the air passage 212, and the nozzle 22 communicates with the chamber 210 through the liquid medicine releasing hole 213. Additionally, the tube 21 includes a liquid medicine discharging plate 214 disposed on the bottom side of the chamber 210, and the liquid medicine discharging plate 214 is aligned with edge of the liquid medicine releasing hole 213. Accordingly, the atomized liquid medicine in the chamber 210 flows to the liquid medicine releasing hole 213 and enters the nozzle 22 by the guiding of the liquid medicine discharging plate 214.

In this embodiment, the nozzle tube 20 further includes an atomizing sheet 25. The atomizing sheet 25 is arranged on one side of the liquid medicine releasing hole 213 to filter objects such as impurities flowing into the chamber 210 from the nozzle 22 when the user exhales. In addition, the atomizing sheet 25 also has the functions of vibrating atomization and preventing the liquid medicinal from outflowing.

Moreover, the detecting structure 23 includes a shutter 231, a swinging member 232 and a positioning seat 233 both connected with the shutter 231. The shutter 231 is disposed corresponding to the position of the sensor 13. The shutter 231 includes an extension arm 2311 and a shielding plate 2312. The positioning seat 233 is combined on the tube 21. Moreover, the swinging member 232 includes a rotating shaft 2321 and at least one blade 2322 connected to the rotating shaft 2321. The rotating shaft 2321 is rotatably disposed on the positioning seat 233, and the at least one blade 2322 protrudes from the positioning seat 233. Additionally, one end of the extension arm 2311 is connected to the rotating shaft 2321, and the other end of the extension arm 2311 is connected to the shielding plate 2312 and protrudes from the positioning seat 233 to be positioned above the sensor 13.

It should be noted that the blade 2322 is a thin sheet made of a soft material, and the blade 2322 is blown by air to rotate and swing around the rotating shaft 2321 as the center point.

Specifically, the tube 21 includes a pair of hooks 215 disposed on the liquid medicine discharging plate 214 and located outside the chamber 210. The positioning seat 233 is combined in the exhaust chamber 211 through the pair of hooks 215.

It is worth noticing that the nozzle tube 20 further includes a conductive elastic sheet 26. The conductive elastic sheet 26 is arranged on the bottom of the tube 21. Furthermore, when the host 10 is assembled on the bottom side of the nozzle tube 20, the conductive terminals 122 of the host 10 are electrically connected to the conductive elastic pieces 26.

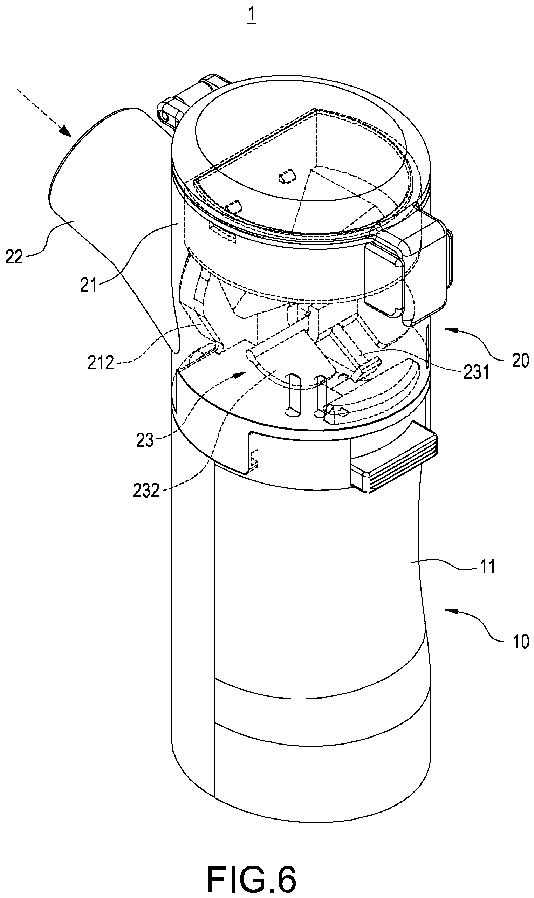

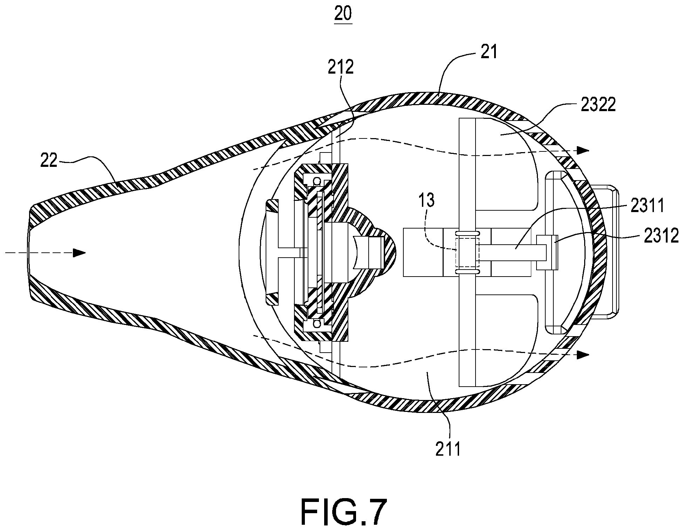

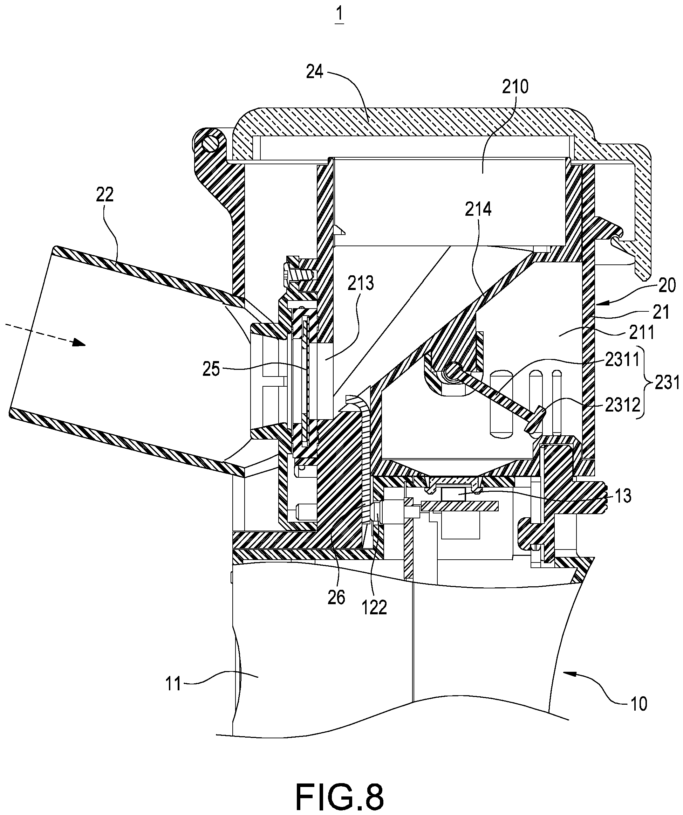

Please further refer to FIG 6 to FIG 8, which depict a schematic view illustrating operation of the nebulizer in exhalation in this disclosure, a schematic view of the air flow path of the nebulizer in exhalation, and a cross sectional view of the nebulizer in exhalation in this disclosure. As shown in the figures, when the user exhales, the air blown from the nozzle 22 flows into the tube 21 and blows the swinging member 232 to drive the shutter 231 to activate the sensor 13. In more detail, when the user exhales, the air blown from the nozzle 22 flows into the exhaust chamber 211 from the air passages 212 on both sides of the tube 21 and blows the swinging member 232. Then, the blade 2322 swings around the rotating shaft 2321 and drives the shutter 231 to rotate. Additionally, the shielding plate 2312 of the shutter 231 leaves the sensing range of the sensor 13 to activate the sensor 13 and send out a sensing signal.

It should be noted that the sensing signal sent from the sensor 13 is transmitted to the sensing circuit board 121, and further the signal is transmitted to the control module 12 for detecting the user's exhalation. Accordingly, when the sensor 13 detects the user's exhalation, the control module 12 stops atomizing the liquid medicinal. Therefore, waste of liquid medicinal is avoided and the effect of administering medication is improved.

## Claims

1. A nebulizer (1), comprising:
a host (10), comprising a main body (11), a control module (12) disposed in the main body (11) and a sensor (13) electrically connected with the control module (12); and
a nozzle tube (20), comprising a tube (21), a nozzle (22) and a detecting structure (23) disposed in the tube (21);
wherein the tube (21) comprises a chamber (210), the nozzle (22) is arranged on one side of the tube (21) and communicates with the chamber (210);
the detecting structure (23) comprises a shutter (231) and a swinging member (232) connected with the shutter (231), and the shutter (231) is disposed corresponding to a position of the sensor (13); and
air blown from the nozzle (22) flows into the tube (21) to blow the swinging member (232) to drive the shutter (231) to activate the sensor (13);
**characterized in that**
the detecting structure (23) further comprises a positioning seat (233) combined on the tube (21); wherein
the swinging member (232) comprises a rotating shaft (2321) and at least one blade (2322) connected to the rotating shaft (2321); and
the rotating shaft (2321) is rotatably disposed on the positioning seat (233), and the at least one blade (2322) protrudes from the positioning seat (233).

2. The nebulizer (1) according to claim 1, wherein the control module (12) comprises a sensing circuit board (121) and a plurality of conductive terminals (122), and the sensor (13) is arranged on the sensing circuit board (121) and electrically connected to the conductive terminals (122).

3. The nebulizer (1) according to claim 2, wherein the control module (12) further comprises a controlling circuit board (123) and a plurality of buttons (124), and the controlling circuit board (123) is electrically connected to the plurality of buttons (124) and the sensing circuit board (121).

4. The nebulizer (1) according to claim 1, wherein the nozzle tube (20) further comprises a top cover (24), and the top cover (24) is movably combined with the tube (21) and covers the chamber (210).

5. The nebulizer (1) according to claim 1, wherein the tube (21) comprises an exhaust chamber (211) and an air passage (212); and
the detecting structure (23) is arranged in the exhaust chamber (211), and the nozzle (22) communicates with the exhaust chamber (211) through the air passage (212).

6. The nebulizer (1) according to claim 5, wherein the tube (21) comprises a liquid medicine releasing hole (213), and the nozzle (22) communicates with the chamber (210) through the liquid medicine releasing hole (213); and
the tube (21) comprises a liquid medicine discharging plate (214) disposed on a bottom side of the chamber (210) and aligned with edge of the liquid medicine releasing hole (213).

7. The nebulizer (1) according to claim 6, wherein the nozzle tube (20) further comprises an atomizing sheet (25) arranged on one side of the liquid medicine releasing hole (213).

8. The nebulizer (1) according to any of the preceding claims, wherein the shutter (231) comprises an extension arm (2311) and a shielding plate (2312); and
one end of the extension arm (2311) is connected to the rotating shaft (2321), and another end of the extension arm (2311) is connected to the shielding plate (2312) and protrudes from the positioning seat (233) to be positioned above the sensor (13).

9. The nebulizer (1) according to any of the preceding claims, wherein the tube (21) comprises a pair of hooks (215) disposed on the liquid medicine discharging plate (214) and located outside the chamber (210), and the positioning seat (233) is combined in the exhaust chamber (211) through the pair of hooks (215).

10. The nebulizer (1) according to any of the preceding claims, wherein the at least one blade (2322) is a thin sheet made of a soft material.

## Patentansprüche

1. Zerstäuber (1), umfassend:
ein Hauptgehäuse (10), das einen Hauptkörper (11), ein in dem Hauptkörper (11) angeordnetes Steuermodul (12) und einen mit dem Steuermodul (12) elektrisch verbundenen Sensor (13) umfasst; und
ein Düsenrohr (20), das ein Rohr (21), eine Düse (22) und eine in dem Rohr (21) angeordnete Erfassungsstruktur (23) umfasst;
wobei das Rohr (21) eine Kammer (210) aufweist, die Düse (22) auf einer Seite des Rohrs (21) angeordnet ist und mit der Kammer (210) in Verbindung steht;
die Erfassungsstruktur (23) einen Verschlusskörper (231) und ein mit dem Verschlusskörper (231) verbundenes schwenkbares Element (232) umfasst, und der Verschlusskörper (231) entsprechend einer Position des Sensors (13) angeordnet ist; und
von der Düse (22) ausgeblasene Luft in das Rohr (21) strömt, um das schwenkbare Element (232) anzuströmen, um den Verschlusskörper (231) anzutreiben, um den Sensor (13) zu aktivieren;
**dadurch gekennzeichnet, dass**
die Erfassungsstruktur (23) ferner einen Positionierungssitz (233) umfasst, der mit dem Rohr (21) zusammengefügt ist, wobei
das schwenkbare Element (232) eine Drehachse (2321) und mindestens ein mit der Drehachse (2321) verbundenes Plättchen (2322) umfasst; und
die Drehachse (2321) drehbeweglich auf dem Positionierungssitz (233) angeordnet ist und das mindestens eine Plättchen (2322) aus dem Positionierungssitz (233) herausragt.

2. Zerstäuber (1) nach Anspruch 1, wobei das Steuermodul (12) eine Sensorschaltungsplatine (121) und eine Vielzahl von leitenden Anschlüssen (122) umfasst und der Sensor (13) auf der Sensorschaltungsplatine (121) angeordnet und elektrisch mit den leitenden Anschlüssen (122) verbunden ist.

3. Zerstäuber (1) nach Anspruch 2, wobei das Steuermodul (12) ferner eine Steuerschaltungsplatine (123) und eine Mehrzahl von Tasten (124) umfasst und die Steuerschaltungsplatine (123) elektrisch mit der Mehrzahl von Tasten (124) und der Sensorschaltungsplatine (121) verbunden ist.

4. Zerstäuber (1) nach Anspruch 1, wobei das Düsenrohr (20) ferner eine obere Abdeckung (24) aufweist und die obere Abdeckung (24) beweglich mit dem Rohr (21) verbunden ist und die Kammer (210) abdeckt.

5. Zerstäuber (1) nach Anspruch 1, wobei das Rohr (21) eine Auslasskammer (211) und einen Luftdurchlass (212) umfasst; und
die Erfassungsstruktur (23) in der Auslasskammer (211) angeordnet ist und die Düse (22) über den Luftdurchlass (212) mit der Auslasskammer (211) in Verbindung steht.

6. Zerstäuber (1) nach Anspruch 5, wobei das Rohr (21) eine Flüssigmedikamenten-Abgabeöffnung (213) aufweist und die Düse (22) über die Flüssigmedikamenten-Abgabeöffnung (213) mit der Kammer (210) in Verbindung steht; und
das Rohr (21) eine Flüssigmedikamenten-Abgabeplatte (214) umfasst, die an einer Bodenseite der Kammer (210) angeordnet und mit dem Rand der Flüssigmedikamenten-Abgabeöffnung (213) ausgerichtet ist.

7. Zerstäuber (1) nach Anspruch 6, wobei das Düsenrohr (20) ferner eine Zerstäubungsplatte (25) umfasst, die auf einer Seite der Flüssigmedikamenten-Abgabeöffnung (213) angeordnet ist.

8. Zerstäuber (1) nach einem der vorhergehenden Ansprüche, wobei der Verschlusskörper (231) einen Verbindungsarm (2311) und eine Abschirmplatte (2312) umfasst; und
ein Ende des Verlängerungsarms (2311) mit der Drehachse (2321) verbunden ist und ein anderes Ende des Verlängerungsarms (2311) mit der Abschirmplatte (2312) verbunden ist und aus dem Positionierungssitz (233) herausragt, um oberhalb des Sensors (13) positioniert zu sein.

9. Zerstäuber (1) nach einem der vorhergehenden Ansprüche, wobei das Rohr (21) ein Paar von Haken (215) aufweist, die an der Flüssigmedikamenten-Abgabeplatte (214) angeordnet sind und sich außerhalb der Kammer (210) befinden, und der Positionierungssitz (233) durch das Paar von Haken (215) mit der Auslasskammer (211) verbunden ist.

10. Zerstäuber (1) nach einem der vorhergehenden Ansprüche, wobei das mindestens eine Plättchen (2322) ein dünnes Plättchen aus einem weichen Material ist.

## Revendications

1. Un nébuliseur (1), comprenant :
un hôte (10), comprenant un corps principal (11), un module de commande (12) disposé dans le corps principal (11) et un capteur (13) connecté électriquement au module de commande (12) ; et
un tube de buse (20), comprenant un tube (21), une buse (22) et une structure de détection (23) disposée dans le tube (21) ;
dans lequel le tube (21) comprend une chambre (210), la buse (22) est disposée sur un côté du tube (21) et communique avec la chambre (210) ;
la structure de détection (23) comprend un obturateur (231) et un élément oscillant (232) relié à l'obturateur (231), et l'obturateur (231) est disposé de manière à correspondre à une position du capteur (13) ; et
l'air soufflé par la buse (22) s'écoule dans le tube (21) pour souffler l'élément oscillant (232) afin d'entraîner l'obturateur (231) et d'activer le capteur (13) ;
**caractérisé par le fait que**
la structure de détection (23) comprend en outre un siège de positionnement (233) combiné au tube (21) ; dans lequel
l'élément oscillant (232) comprend un arbre rotatif (2321) et au moins une lame (2322) reliée à l'arbre rotatif (2321) ; et
l'arbre rotatif (2321) est disposé de manière rotative sur le siège de positionnement (233), et au moins une lame (2322) fait saillie du siège de positionnement (233).

2. Le nébuliseur (1) selon la revendication 1, dans lequel le module de commande (12) comprend une carte de circuit imprimé de détection (121) et une pluralité de bornes conductrices (122), et le capteur (13) est disposé sur la carte de circuit imprimé de détection (121) et connecté électriquement aux bornes conductrices (122).

3. Le nébuliseur (1) selon la revendication 2, dans lequel le module de commande (12) comprend en outre une carte de circuit de commande (123) et une pluralité de boutons (124), et la carte de circuit de commande (123) est connectée électriquement à la pluralité de boutons (124) et à la carte de circuit de détection (121).

4. Le nébuliseur (1) selon la revendication 1, dans lequel le tube de buse (20) comprend en outre un couvercle supérieur (24), et le couvercle supérieur (24) est combiné de manière mobile avec le tube (21) et recouvre la chambre (210).

5. Le nébuliseur (1) selon la revendication 1, dans lequel le tube (21) comprend une chambre d'échappement (211) et un passage d'air (212) ; et.
la structure de détection (23) est disposée dans la chambre d'échappement (211), et la buse (22) communique avec la chambre d'échappement (211) à travers le passage d'air (212).

6. Le nébuliseur (1) selon la revendication 5, dans lequel le tube (21) comprend un trou de libération de médicament liquide (213), et la buse (22) communique avec la chambre (210) à travers le trou de libération de médicament liquide (213) ; et.
le tube (21) comprend une plaque de déchargement du médicament liquide (214) disposée sur la face inférieure de la chambre (210) et alignée sur le bord du trou de libération de médicament liquide (213).

7. Le nébuliseur (1) selon la revendication 6, dans lequel le tube de buse (20) comprend en outre une feuille d'atomisation (25) disposée sur un côté du trou de libération de médicament liquide (213).

8. Le nébuliseur (1) selon l'une quelconque des revendications précédentes, dans lequel l'obturateur (231) comprend un bras d'extension (2311) et une plaque de blindage (2312) ; et.
une extrémité du bras d'extension (2311) est reliée à l'arbre rotatif (2321), et une autre extrémité du bras d'extension (2311) est reliée à la plaque de blindage (2312) et fait saillie du siège de positionnement (233) pour être positionnée au-dessus du capteur (13).

9. Le nébuliseur (1) selon l'une quelconque des revendications précédentes, dans lequel le tube (21) comprend une paire de crochets (215) disposés sur la plaque de déchargement du médicament liquide (214) et situés à l'extérieur de la chambre (210), et le siège de positionnement (233) est combiné dans la chambre d'échappement (211) par l'intermédiaire de la paire de crochets (215).

10. Le nébuliseur (1) selon l'une quelconque des revendications précédentes, dans lequel la au moins une lame (2322) est une feuille mince en matériau souple.
